# EUROPEAN PATENT APPLICATION

(11) **EP 1 506 792 A2**
(43) Date of publication of application: **16.02.2005**
(21) Application number: 04007557.4
(22) Date of filing: 29.03.2004
(51) Int. Cl.: A61M 15/00, A61F 9/00

(54) **Liquid medicament delivery system**

(30) Priority: 14.08.2003 US 640058
(71) Applicant: Hughes, Nathaniel, California 92264 (US); SHAW, Leon, California 90405 (US)
(72) Inventor: Hughes, Nathaniel, California 92264 (US); SHAW, Leon, California 90405 (US)
(74) Representative: Modiano, Guido, Dr.-Ing.

(57) **Abstract**

A liquid medicament delivery system utilizing a carrier for a dose of liquid medicament. A conduit having an entrance and exit communicates with the carrier to permit the same to travel along the conduit. A propellant and the liquid medicament carried by the conduit are formed into stable vortex eddy and passed to a mixing chamber where the medicament is delivered through an outflow aperture for use.

## Description

The present invention relates to a novel and useful liquid medicament delivery system.

Medicines delivered to a patient are often in the form of liquids. For example, eye drops are topically applied to the eye. Other liquid medicinal solutions are inhaled for use in the nasal passages, lungs and the like.

In the past, delivery of liquid drugs have involved a dropper or devices which squirt the liquid to the anatomical region where it is needed. These liquid solutions, are often wasted, to a large degree, and inaccurately delivered such that the liquid solution undesirably spreads over the clothing or skin of the user. Often the result is that an inadequate amount of solution is delivered to the anatomy of a patient such as an eyeball due to inefficient delivery procedures.

The use of an eyecup in the case where a liquid medicament is delivered to an eyeball does not wholly solve the problem of delivery of adequate amounts of liquid drugs. It is desirous that a liquid medicament not only coat the front part of the eyeball but that the back surface of the eyeball should be coated as well an eyecup does not achieve this end.

A liquid medicament delivery system which is efficient and accurate would be a notable advance in the medical field.

### BRIEF SUMMARY OF THE INVENTION

In accordance with the present invention a novel and useful liquid medicament delivery system is herein provided.

The system of the present invention utilizes a carrier of a dose of liquid medicament. The carrier may take the form of a plate having a container with a chamber attached thereto. The medicament is sealed into the chamber by a membrane which is penetrable by a probe such as a cannula. The carrier, of course, would contain an accurate dose of liquid medicament and is maintained in a protected and sterile environment until used.

A conduit or cannula is also included in the present invention. The conduit includes an entrance and an exit for the liquid medicament and is capable of communicating with the chamber of the carrier to lead the chamber of the carrier to lead the liquid medicament from the chamber to the exit of the conduit. The conduit may be mounted on a plate and include a tip which is capable of penetrating the membrane of the carrier. A source of propellant is also employed in the present invention to motivate the movement of the liquid medicament from the carrier to the ultimate user. The source of propellant would be a non-toxic bio-compatible material which may be in an aerosol form.

A mixing chamber is also utilized in the present invention to combine the propellant with the liquid medicament flowing through the conduit. The mixing chamber may also include a nozzle for generating a stable vortex eddy field. In this manner, the propellant and liquid medicament are formed into a homogenized mixture. The mixing chamber includes an outflow aperture which guides the propellant and liquid medicament to the user-patient. The outflow aperture of the mixing chamber may take many forms such as one that is oval in cross-sectional configuration, one that is in the form of an eyecup, and the like. The outflow aperture may be constructed of elements that are interchangeable to produce this result.

It may be apparent that a novel and useful liquid medicament delivery system has been hereinabove described.

It is therefore an object of the present invention to provide a liquid medicament delivery system which is capable of delivering medicinal solutions at extremely low velocities in an aerosol fog to an anatomical portion of the body such as the surface of an eye.

Another object of the present invention is to provide a liquid medicament delivery system which is capable of delivering very accurate masses of liquid medicament to an eye and in relatively small quantities.

A further object of the present invention is to provide a liquid medicament delivery system which is comfortable to the patient and very carefully controls the delivery of liquid medicament solutions in a focused manner.

A further object of the present invention is to provide a liquid medicament delivery system which may deliver a total dose of a medicinal solution, obviating the need for repeated applications of the same.

Yet another object of the present invention is to provide a liquid medicament delivery system which utilizes propellants that are safe and are only required in minimal quantities per dose of liquid medicament delivered.

A further object of the present invention is to provide a liquid medicament delivery system which is capable of producing an aerosol with a propellant and operates in conjunction with liquid medicaments possessing large variations of density and/or viscosity.

Another object of the present invention is to provide a liquid medicament delivery system which is efficient in reaching surfaces of the eye not achieved by prior art devices.

Another object of the present invention is to provide a liquid medicament delivery system which employs the creation of a staple vortex eddy field to produce an aerosol fog which uniformly distributes the liquid medicament for use by a patient.

Another object of the present invention is to provide a liquid medicament delivery system which is versatile in that hardware components may be transformed quickly to deliver liquid medicaments to various portions of the anatomy such as the eye, the mouth, and the like.

A further object of the present invention is to provide a liquid medicament delivery system which provides sterile drug loading and delivery and includes disposable portions.

The invention possesses other objects and advantages especially as concerns particular characteristics and features thereof which will become apparent as the specification continues.

### BRIEF DESCRIPTION OF THE SEVERAL VIEWS OF THE DRAWING

Fig. 1 is a side elevational view of the system of the present invention.
Fig. 2. is a sectional view taken along line 2-2 of Fig.
Fig. 3 is a side elevational view of an eyecup which may be used in substitution for the oval aperture article of Fig. 2.
Fig. 4 is a side elevational view of the liquid medicament carrier.
Fig. 5 is a front elevational view of the liquid medicament carrier.
Fig. 6 is a top plan view of the conduit and holder used in the present invention.
Fig. 7 is a magnified sectional view of the tip of the conduit depicted in Fig. 6.
Fig. 8 is a bottom plan view of the system of the present invention depicting the conduit plate in place and in exploded configuration.
Fig. 9 is a partial right side elevational view of the system depicted in Fig. 1 showing the guide for the conduit plate.
Fig. 10 is a sectional view taken along line 10-10 of Fig. 1 depicting the vortex generator.

For a better understanding of the invention reference is made to the following detailed description of the preferred embodiments thereof which should be taken in conjunction with the prior described drawings.

### DETAILED DESCRIPTION OF THE PREFERRED EMBODIMENTS OF THE INVENTION

Various aspects of the present invention will evolve from the following detailed description of the preferred embodiments thereof which should be referenced to the hereinabove delineated drawings.

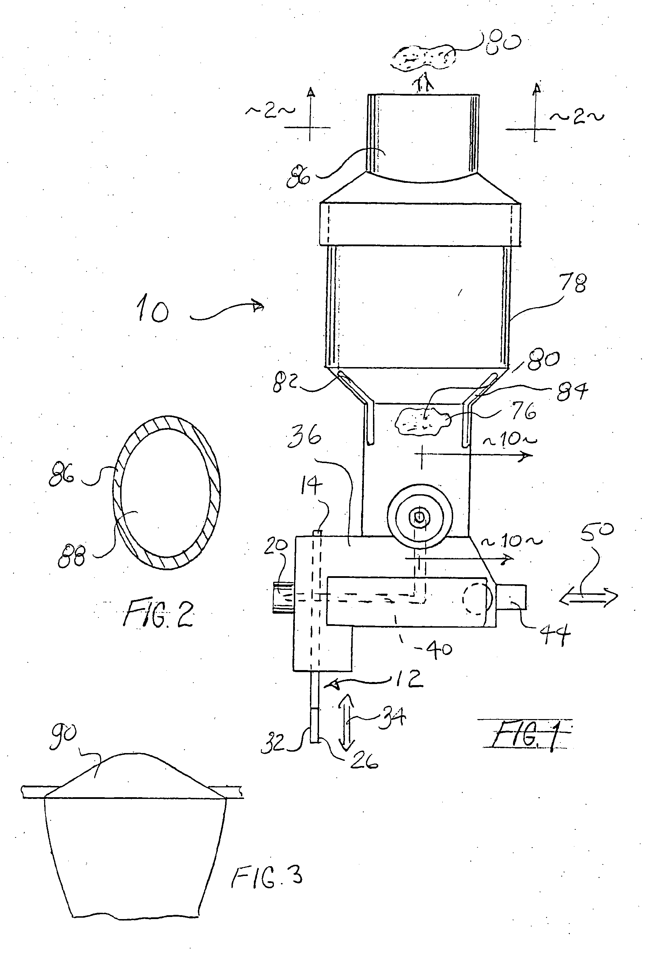

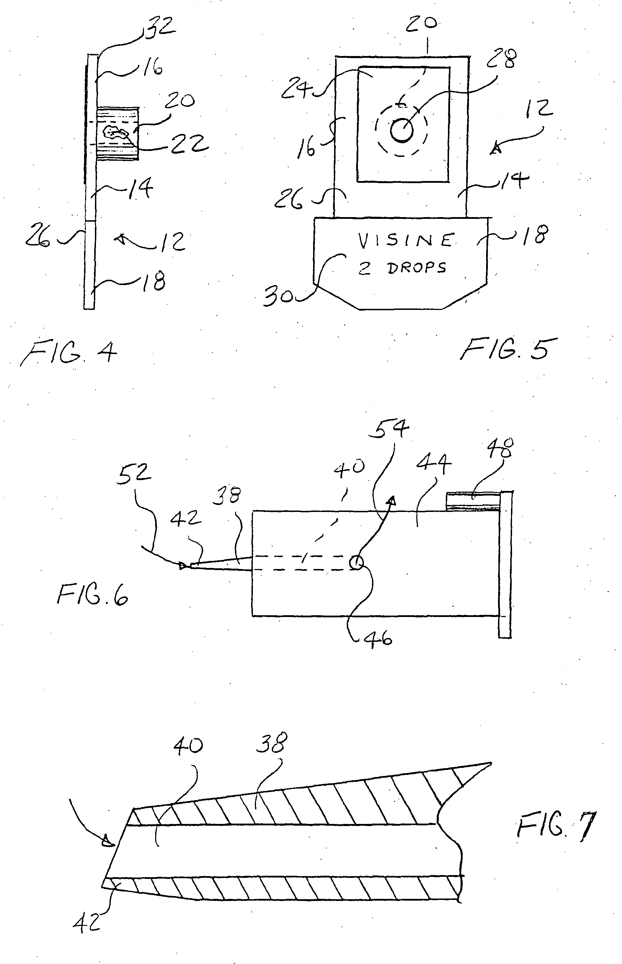

A preferred embodiment of the invention is shown in the drawings by reference character 10. Delivery system 10 includes as one of its elements a carrier 12 for liquid medicament. Turning to Figs. 4 and 5, it may be observed that carrier 12 includes a plate 14 having an upper portion 16 and a lower portion, 18. upper portion 16 of plate 14 includes a container 20 for a particular dose of the liquid medicament to be delivered by system 10. Chamber 22 within container 20 confines such dose of liquid medicament. A seal strip 24 on side 26 of plate 14 encloses chamber 22 of container 20. It should be apparent that opening 28 to chamber 22 is depicted in Fig. 5 since seal strip 24 has been punctured or penetrated. Lower portion 18 includes indicia 30 identifying the liquid contents of chamber 22. Very precise quantities of liquid medicament may be stored in chamber 22 of container 20 and delivered through system 10 by this expedient. Container 20 extends from side 32 of plate 14 and is visible on Fig. 1. Directional arrow 34 indicates the movement of carrier 12 into and out of housing 36.

System 10 also is formed with a conduit 38 having a passageway 40, Figs. 1, 6, and 7. With reference to Fig. 6 in particular, conduit 38 is shown In the form of a cannula having a convergent tip 42. Conduit or cannula 38 is fastened within a sliding holder 44 and includes an orifice 46 to allow the passage of liquid medicament from chamber 22 of container 20. Such movement of liquid medicament occurs when cannula 38 penetrates seal strip 24 within housing 36. The movement of sliding holder 44 is either controlled by stop 48 or by the bottoming of tip 42 of cannula 48 within container 20. Directional arrow 50, Fig. 1, indicates the in and out movement of holder 44 and penetrating conduit or cannula 38. Directional arrows 52 and 54, Fig. 6, show the movement of liquid medicament through conduit 38 and from orifice 46.

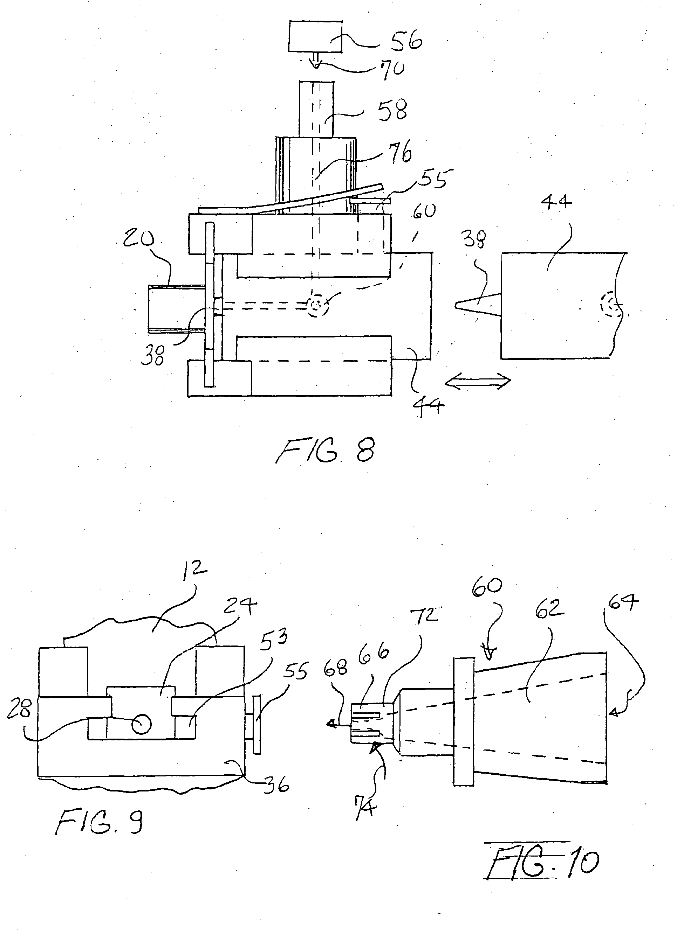

Referring to Fig. 9, guide slot 53 of housing 36 directs the movement of sliding holder 44 into its position for penetration of seal strip 24. Button 55 frictionally engages the side of holder 44, when pressed, to maintain its position within housing 36 as depicted in Fig. 1. It should be noted that holder 44 is also shown within housing 36 and apart from 36 in Fig. 8 to emphasize its movement within guide slot 53.

A source of propellant 56, Fig. 8 is also found in the present invention and may be of conventional origin. Any non-toxic bio-compatible gas may be employed as source 56. Source 56 is sent into nipple 58 and is formed into a stable vortex eddy field by vortex generator 60, Fig. 10. That is to say, the liquid medicament enters the vortex generator converging chamber 62, directional arrow 64, and exits the same through end portion 66, directional arrow 68. Propellant from source 56 enters nipple 58, directional arrow 70, Fig. 8, and hits the grooved surface 72 of end portion 66 of generator 60. The eddy field of the vortex then draws liquid medicament through chamber 62. Directional arrow 68 indicates the movement of liquid medicament and propellant from vortex generator 60. Directional arrow 74 indicates the movement of propellant through passageway 76 within housing 34.

Mixing chamber 76 is found within vessel 78 which is connected to housing 36. Propellant and liquid medicament are mixed within chamber 76 and indicated as an aerosol 80. Aerosol 80 is relatively slow moving and homogenous. Slits 82 and 84 allow outside air to enter chamber 76 to aid in the smooth formation of the aerosol 80 in chamber 76.

After being passed through chamber 76 of vessel 78, the aerosol 80 exits end fitting 86. Referring again to Fig. 1, it may be observed that the outflow aperture 88 of end fitting 86 is oval in configuration and may be best suited for oral administration of liquid medicament. Fig. 3 shows a side view of an eyecup 90 which may be used instead of end fitting 86 to deliver aerosol 80 to an eye.

In operation, the user loads carrier 12 into housing 36 by sliding the same into the position depicted in Fig. 1. Container 20 is then aligned with cannula or conduit 38 which slides within guide 53 to penetrate seal strip 24. Such penetration releases the liquid medicament within chamber 22 of container 20. The liquid medicament then flows through conduit 40 and into mixing chamber 76 via converging chamber 62 of vortex generator 60. Propellant from propellant source 56 passes through nipple 58 and into the exterior end portion 66 of vortex generator 60 where the grooved surface 66 .generates a stable eddy field. Directional arrow 68. represents the mixture of propellant and liquid medicament which is passed through vortex generator 60. The aerosol mixture within mixing chamber 76 of vessel 78 then passes through end fitting 86 or 90 for use. It has been found that the system of the present invention is highly efficient and employs virtually all of the dose of liquid medicament loaded into chamber 22 of container 20 found on carrier 12.

While in the foregoing, embodiments of the present invention have been set forth in considerable detail for the purposes of making a complete disclosure of the invention, it may be apparent to those of skill in the art that numerous changes may be made in such detail without departing from the spirit and principles of the invention.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A liquid medicament delivery system, comprising:
a. a carrier of an dose of liquid medicament;
b. a conduit including an entrance and an exit for the liquid medicament, said conduit communicating with said carrier;
c. a source of propellant; and
d. a mixing chamber communicating with the exit of said conduit and said source of propellant, said mixing chamber including an outflow aperture for guiding said propellant and liquid medicament to a user.

2. The system of claim 1 in which said carrier comprises an element having a chamber for containing a.dose of liquid medicament.

3. The system of claim 2 in which said conduit entrance is sized to enter said chamber of said carrier.

4. The system of claim 1 in which said conduit entrance comprises an end portion having a tapered surface.

5. The system of claim 1 which additionally comprises a carrier guide for sliding engagement of said carrier.

6. The system of claim 5 in which said carrier comprises an element having a chamber for containing a dose of liquid medicament.

7. The system of claim 6 in which said conduit entrance is sized to enter said chamber of said carrier.

8. The system of claim 5 in which said conduit entrance comprises an end portion having a tapered surface.

9. The system of claim 1 which additionally comprises a support for said conduit and a conduit guide, said conduit guide positioning said conduit entrance in communication with said carrier and said conduit exit in communication with said mixing chamber.

10. The system of claim 9 in which said carrier comprises an element having a chamber for containing a dose of liquid medicament.

11. The system of claim 10 in which said conduit entrance is sized to enter said chamber of said carrier.

12. The system of claim 9 in which said conduit entrance comprises an end portion having a tapered surface.

13. The system of claim 9 which additionally comprises a carrier guide for sliding engagement of said carrier.

14. The system of claim 4 in which said conduit exit includes an end with a mitered edge surface.

15. The system of claim 1 in which additionally comprises a member having an oval cross sectional configuration, said member communicating with said outflow aperture of said mixing chamber.

16. The system of claim 1 which additionally comprises cup-shaped member, said cup-shaped member communicating with said outflow aperture of said mixing chamber.

17. The system of claim 1 which additionally comprises a vortex generator located in the vicinity of said entrance to said mixing chamber.
